# EUROPEAN PATENT SPECIFICATION

(11) **EP 2 745 864 B1**
(45) Date of publication and mention of the grant of the patent: **18.01.2017**
(21) Application number: 12198329.0
(22) Date of filing: 20.12.2012
(51) Int. Cl.: A61M 1/36

(54) **Blood treatment apparatus, control method and computer program**
Blutbehandlungsvorrichtung, Steuerungsverfahren und Computerprogramm
Appareil de traitement sanguin, procédé de commande et programme informatique

(43) Date of publication of application: 25.06.2014
(73) Proprietor: Gambro Lundia AB, S-220 10 Lund (SE)
(72) Inventor: Suffritti, Mauro, 41036 Medolla (IT); Ramicone, Valerio, 67039 Sulmona (IT)
(74) Representative: Ponzellini, Gianmarco

(56) References cited:
- EP-A1- 2 535 067
- US-A1- 2005 131 331
- US-A1- 2006 079 826
- US-A1- 2011 272 337

## Description

### Technical field

The present invention generally relates to a blood treatment apparatus, a control method for the apparatus, and a computer program for implementing the control method. In particular, the invention relates to an approach for emptying blood lines of the blood treatment apparatus from blood.

### Background

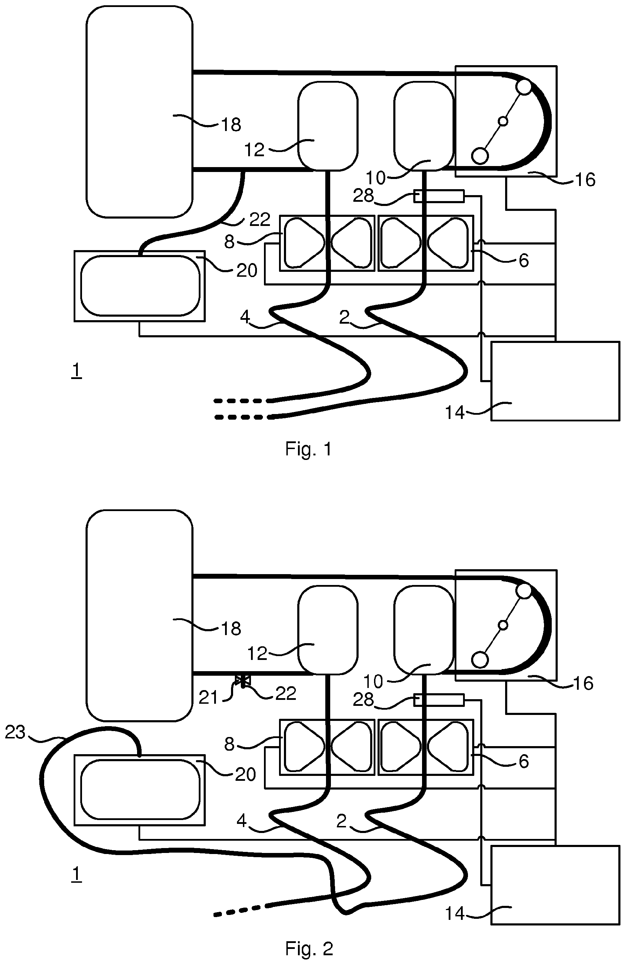

Blood treatments apparatuses, such as hemodialysis apparatuses, collect blood from a patient, treat the blood and returns it to the patient. Consequently, the patient needs to be connected to the apparatus during treatment. Fig. 1 schematically illustrates an apparatus 1 for extracorporeal blood treatment during treatment operations according to these traditional operations. The apparatus 1 comprises an arterial line 2 and a venous line 4 for connecting to a patient. An arterial clamp 6 can be arranged to selectably clamp or unclamp the arterial line 2. The arterial line 2 is connected to an arterial chamber 10, and the venous line 4 is connected to a venous chamber 12. Here, the terms "venous" and "arterial" in connection with "clamp", "line" and "chamber" are used due to their association at connection to a patient, and should not be construed to be a part of the blood vessels of the patient. The arterial and venous chambers 10, 12 have the purpose of forming a buffer for the blood such that the arterial and venous lines 2, 4 are blood filled during operation. A blood pump 16, e.g. a peristaltic pump, is connected to the arterial chamber 10 and arranged to provide blood from the arterial chamber 10 via a blood treatment device 18 towards the venous chamber 12. There is further an infusion liquid port 20 arranged to provide infusion liquid from an infusion liquid source via an infusion liquid input 22, which can be arranged at a line between the blood treatment device 18 and the venous chamber 12. The venous line 4 can also be provided with a venous clamp 8 for selectably clamping or unclamping the venous line 4. One or more sensors, e.g. a pressure sensor 28 on the arterial line 2, can also be provided in the apparatus 1. The apparatus 1 can also comprise a controller 14 for controlling any controllable element 6, 8, 16, 20 of the apparatus 1 and receive measurement signals from sensors 28 of the apparatus 1. The connection to the patient is thus is performed using the arterial and venous lines 2, 4, which can be connected to respective needles at the patient. When the treatment is ready, the patient evidently should be disconnected. For proper disconnection, a blood restitution procedure is performed. Fig. 2 schematically illustrates the traditional apparatus 1 for extracorporeal blood treatment during blood restitution operations. Normally, the operator disconnects the arterial line 2 from the patient and connects the arterial line to a saline solution source line 23, which is connected to e.g. a saline solution bag or a saline solution port 20 of a saline solution source line of the clinic. The connection from the infusion liquid port 20 to the infusion liquid input 22 is normally removed, and the infusion liquid input is closed, e.g. clamped by a clamp 21. The blood restitution procedure is then performed by running a pump of the apparatus for restitution of the blood to the patient. Thereafter, the venous line is disconnected. Thus, a number of operations, first with at the patient, then at the apparatus, and then at the patient again are performed. Furthermore, the operator risks coming into contact with the patient's blood when disconnecting the arterial line, which can be seen as a security issue.

Prior art document US 2006/079826 discloses a method for the return of blood from a blood treatment apparatus. A substituate pump contained in the treatment apparatus displaces the blood by means of correspondingly transported substituate fluid until it is detected in corresponding detectors that substituate fluid is flowing back in the line instead of blood. From this moment in time, the blood volume is further displaced in a controlled manner until it has reached the line outlet of the corresponding line.

US 2011/0272337 discloses a method of blood restitution wherein saline solution is introduced into the arterial line upstream of the blood pump. In a first phase, an arterial clamp is closed and the blood in the venous line is returned to the patient. After a predetermined volume of saline solution has been introduced into the arterial line, the blood pump is stopped, the arterial clamp is opened, and blood in the arterial line is flushed back to the patient.

There is a desire to provide an efficient and secure disconnection.

### Summary

An object of the invention, as defined in claims 1 and 10, is to at least alleviate the above stated problem. The
present invention is based on the understanding that by adaptation of the apparatus enables the operator to work more efficient and also be less exposed to the blood contact risk.

According to a first aspect, there is provided a blood treatment apparatus for extracorporeal blood treatment. The apparatus comprises an arterial line and a venous line for connecting to a patient. An arterial clamp is arranged on the arterial line to selectably clamp and unclamp the arterial line. There is further an arterial chamber connected to the arterial line, a venous chamber connected to the venous line, a blood pump connected to the arterial chamber and arranged to provide blood from the arterial chamber via a blood treatment device towards the venous chamber. An infusion liquid port is arranged for connection of a infusion liquid source. A controller is arranged to, at blood restitution operation, control first to fourth phase of the blood restitution operation.

The first phase of the blood restitution operation includes control operations to ascertain that the arterial line is filled with blood, and then to set the arterial line clamp to a clamped state wherein the blood restitution operation is enabled to proceed to a second phase of the blood restitution operation.

The second phase of the blood restitution operation includes control operations to infuse liquid from the infusion liquid port to the arterial chamber and control the blood pump together with the supplied infusion to apply a positive pressure in the arterial line while the arterial line clamp is controlled to be in a clamped state such that blood is restituted, by drive control of the blood pump, to the patient via the venous line. The blood restitution operation is enabled to proceed to a third phase of the blood restitution operation when the blood of the venous line has been restituted to the patient.

The third phase of the blood restitution operation includes control operations to ascertain, by monitoring, that no air is present in the arterial line, wherein the blood restitution operation is enabled to proceed to a fourth phase of the blood restitution operation when it is ascertained that no air is present in the arterial line.

The fourth phase of the blood restitution operation includes control operations to set the arterial line clamp to an unclamped state, and set the blood pump to a blocked state, and then infuse infusion liquid into the arterial chamber such that blood in the arterial line is restituted to the patient.

The control operation to ascertain that the arterial line is filled with blood may include to initially set the arterial line clamp to be in unclamped state, and then control the blood pump to apply a negative pressure in the arterial chamber such that a volume of the arterial line is aspired, and then set the arterial line clamp to a clamped state.

The control operations to ascertain that no air is present in the arterial line may include determination of compression characteristics of the arterial line by a test, with the arterial line clamp in a clamped state, by application of a pressure in the arterial line by control of the blood pump and monitoring of the pressure in the arterial line (102) by a pressure sensor.

The monitoring of the pressure in the arterial line may include a pressure sample before and a pressure sample after changing the volume of the arterial chamber by a predetermined volume aspired by the controlling of the blood pump, and applying Boyle's law for determining whether any air is present in the arterial line.

The infusion of liquid to the arterial chamber may comprise to control an infusion pump, by the controller, to provide the infusion liquid into the arterial chamber via a service line connected to the arterial chamber.

The apparatus may comprise a user interface, UI, controlled by the controller, wherein the UI is arranged to enable a blood restitution button when blood treatment operations are ready, and upon operator actuation of the blood restitution button, the controller is configured to activate the blood restitution operation. The UI may be arranged to, when the blood restitution button is enabled, provide an instruction to the operator to connect an outlet connector from an infusion liquid port to an arterial service line being in connection with the arterial chamber. The UI may comprise a touchscreen, and the blood restitution button may be a soft button presented on the touch screen.

The apparatus may be a hemodialysis apparatus, and the blood treatment device may be a dialyser.

According to a second aspect, there is provided a method of controlling a blood treatment apparatus for extracorporeal blood treatment, where the apparatus comprises an arterial line and a venous line for connecting to a patient, wherein an arterial clamp is arranged on the arterial line for selectable clamping and unclamping of the arterial line, an arterial chamber for connecting to the arterial line, a venous chamber for connecting to the venous line, a blood pump connected to the arterial chamber and for providing blood via a blood treatment device towards the venous chamber, and an infusion liquid port for connection of a infusion liquid source. The method comprises controlling a first to fourth phase of control operations.

The first phase of control operation comprises ascertaining that the arterial line is filled with blood, and then setting the arterial line clamp to a clamped state, and then enabling the operation to proceed to a second phase of the operation.

The second phase of the control operation comprises infusing infusion liquid from the infusion liquid port to the arterial chamber, and controlling the blood pump together with the supplied infusion liquid to apply a positive pressure in the arterial line while the arterial line clamp is in a clamped state such that a liquid flow is achieved, by driving the blood pump, through the venous line, and then enabling the operation to proceed to a third phase of the operation when the blood of the venous line is emptied.

The third phase of the control operation comprises ascertaining, by monitoring, that no air is present in the arterial line, and enabling the operation to proceed to a fourth phase of the operation when it is ascertained that no air is present in the arterial line.

The fourth phase of the control operation comprises setting the arterial line clamp to an unclamped state, and setting the blood pump to a blocked state, and then infusing infusion liquid into the arterial chamber such that blood in the arterial line is emptied.

The ascertaining that the arterial line has been filled with blood may comprise initially setting the arterial line clamp to be in unclamped state, and then controlling the blood pump to apply a negative pressure in the arterial chamber such that a volume of the arterial line is aspired, and then setting the arterial line clamp to a clamped state.

The ascertaining that no air is present in the arterial line may comprise determining compression characteristics of the arterial line by a test, with the arterial line clamp in a clamped state, by applying a pressure in the arterial line by controlling the blood pump and monitoring the pressure in the arterial line. The monitoring of the pressure in the arterial line may be performed before and after changing the volume of the arterial chamber by a predetermined volume aspired by the controlling of the blood pump, and the determining may then comprise applying Boyle's law for determining whether any air is present in the arterial line.

The infusing of liquid to the arterial chamber may comprise providing the infusion liquid into the arterial chamber via a service line connected to the arterial chamber.

The apparatus may comprise a user interface, UI, wherein the method may further comprise enabling a blood restitution button on the UI when blood treatment operations are ready, receiving a signal upon operator actuation of the blood restitution button, and activating, upon reception of the signal, the controlling of the operations. The method may further comprise providing an instruction through the UI, when the blood restitution button is enabled, to the operator to connect an outlet connector from an infusion liquid port to an arterial service line being in connection with the arterial chamber.

The UI may comprise a screen, and the enabling of the blood restitution button comprises presenting the blood restitution button as a soft button on the screen.

According to a third aspect, there is provided a computer program comprising program code means for performing the method according to the second aspect when said program is run on a processor of a controller of a blood treatment apparatus for extracorporeal blood treatment.

According to further aspects, apparatuses, and methods for controlling the apparatuses, for extracorporeal blood treatment are provided, where the apparatus comprising an arterial line and a venous line for connecting to a patient, wherein an arterial clamp is arranged on the arterial line for selectable clamping and unclamping of the arterial line, an arterial chamber for connecting to the arterial line, a venous chamber for connecting to the venous line, a blood pump connected to the arterial chamber and for providing blood via a blood treatment device towards the venous chamber, and an infusion liquid port for connection of a infusion liquid source. The controlling comprises, for the emptying of blood in the arterial line, such as restituting the blood to a patient, controlling one phase of the operation comprising ascertaining, by monitoring, that no air is present in the arterial line, and enabling the operation to proceed to another phase of the operation when it is ascertained that no air is present in the arterial line. Then, controlling of the another phase of the operation comprises setting the arterial line clamp to an unclamped state, and setting the blood pump to a blocked state, and then infusing infusion liquid into the arterial chamber such that blood in the arterial line is emptied.

One or more of the embodiments provides the advantage that the operator of the apparatus will experience that blood restitution process is taken care of by the apparatus, and no re-connections should normally be necessary, thus providing, in addition to efficiency, a safer handling with greatly reduces contamination risks.

One or more of the embodiments provides the advantage that robustness and/or economy of used gear can be improved since use of conventional costly air detectors can be avoided.

Other objectives, features and advantages of the present invention will appear from the following detailed disclosure, from the attached dependent claims as well as from the drawings. Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

### Brief description of the drawings

The above, as well as additional objects, features and advantages of the present invention, will be better understood through the following illustrative and nonlimiting detailed description of preferred embodiments of the present invention, with reference to the appended drawings.
Fig. 1 schematically illustrates a traditional apparatus for extracorporeal blood treatment during treatment operations according to traditional operations.
Fig. 2 schematically illustrates the traditional apparatus for extracorporeal blood treatment during blood restitution operations.
Fig. 3 schematically illustrates structure of an apparatus for extracorporeal blood treatment according to an embodiment.
Fig. 4 is a flow chart illustrating control of an apparatus for extracorporeal blood treatment according to an embodiment.
Fig. 5 schematically illustrates operation of the apparatus for extracorporeal blood treatment according to an embodiment in a part of blood restitution operations.
Fig. 6 schematically illustrates operation of the apparatus for extracorporeal blood treatment according to an embodiment in a part of blood restitution operations.
Fig. 7 schematically illustrates operation of the apparatus for extracorporeal blood treatment according to an embodiment in a part of blood restitution operations.
Fig. 8 schematically illustrates operation of the apparatus for extracorporeal blood treatment according to an embodiment in a part of blood restitution operations.
Fig. 9 is a flow chart illustrating ascertaining that an arterial line is blood filled according to an embodiment.
Fig. 10 is a flow chart illustrating restitution of blood of a venous line according to an embodiment.
Fig. 11 is a flow chart illustrating ascertaining that no air is present in an arterial line according to an embodiment.
Fig. 12 is a flow chart illustrating restitution of blood of an arterial line according to an embodiment.
Fig. 13 is a flow chart illustrating control of a user interface of the apparatus for extracorporeal blood treatment according to an embodiment.
Fig. 14 schematically illustrates a computer-readable medium for storing a computer program for controlling an apparatus for extracorporeal blood treatment, and a processor, to which the computer program can be downloaded and be executed, for control of the apparatus for extracorporeal blood treatment according to an embodiment.

### Detailed description

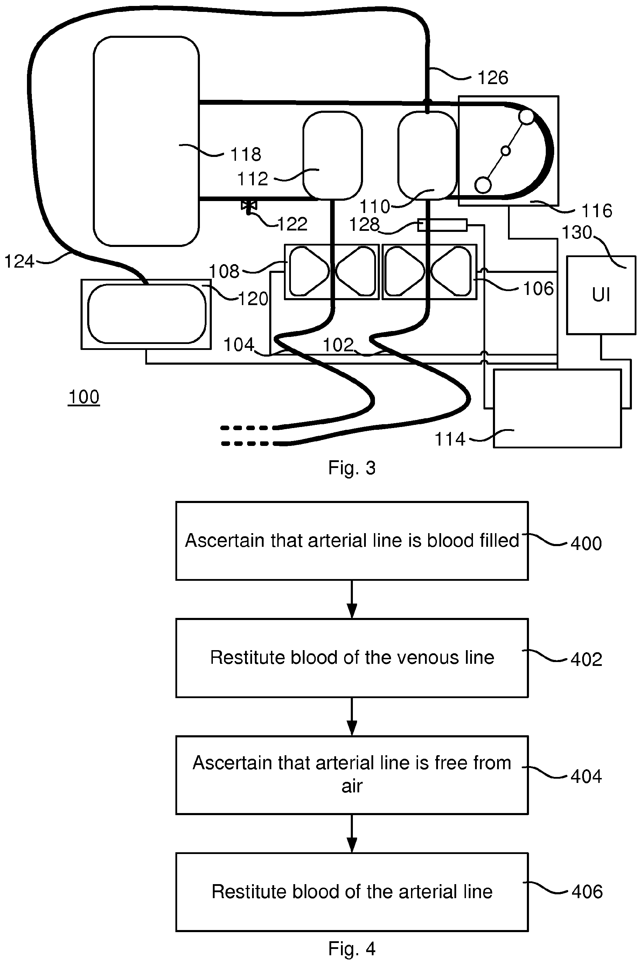

Fig. 3 schematically illustrates structure of an apparatus 100 for extracorporeal blood treatment according to an embodiment. The apparatus 100 comprises an arterial line 102 and a venous line 104 for connecting to a patient. An arterial clamp 106 can be arranged to selectably clamp or unclamp the arterial line 102. The arterial line 102 is connected to an arterial chamber 110, and the venous line 104 is connected to a venous chamber 112. Here, the terms "venous" and "arterial" in connection with "clamp", "line" and "chamber" are used due to their association at connection to a patient, and should not be construed to be a part of the blood vessels of the patient. The arterial and venous chambers 110, 112 have the purpose of forming a buffer for the blood such that the arterial and venous lines 102, 104 are blood filled during operation. A blood pump 116, e.g. a peristaltic pump, is connected to the arterial chamber 110 and arranged to provide blood from the arterial chamber 110 via a blood treatment device 118 towards the venous chamber 112. There is further an infusion liquid port 120 arranged to provide infusion liquid from an infusion liquid source. As in the traditional apparatus 1, the infusion liquid can be provided via an infusion liquid input 122, which can be arranged at a line between the blood treatment device 118 and the venous chamber 112. However, infusion liquid is here provided via an infusion liquid line 124 connected to a service line 126 such that infusion liquid can be provided to the arterial chamber 110. The venous line 104 can also be provided with a venous clamp 108 for selectably clamping or unclamping the venous line 104. One or more sensors, e.g. a pressure sensor 128 on the arterial line 102, can also be provided in the apparatus 100. The apparatus 100 also comprises a controller 114 for controlling any controllable element 106, 108, 116, 120 of the apparatus 100 and receive measurement signals from sensors 128 of the apparatus 100. In particular, the controller 114 is arranged to control the apparatus 100 during blood restitution by applying four phases such that an operator only needs to initiate the blood restitution and the apparatus 100 performs the complete blood restitution without the operator needing to re-connect any of the arterial or venous lines 102, 104 during the restitution, and thereby does not risk coming into contact with the patient's blood. The structure of the apparatus 100 provides for this, and together with the particular control pattern of the apparatus 100 this is made feasible. The control pattern includes that the controller controls controllable elements of the apparatus 100 according to the phases demonstrated below:
During a first phase, the controller ascertains that the arterial line is filled with blood. This can be made by the blood pump 116 is controlled to apply a negative pressure in the arterial chamber 110 while the arterial clamp 106 is in an unclamped state. Thereby, the arterial line 102 is aspired, and this is performed by driving the blood pump 116 to aspire a volume that at least corresponds to the volume of the arterial line 102. Thereafter, the arterial clamp 106 is controlled to be set to a clamped state. This phase may seem superfluous since the arterial line 102 is expected to be blood filled since the treatment that reasonably is made just before the blood restitution. However, the phase have the purpose of ascertaining that no air has managed to enter the arterial line 102 between the treatment and the blood restitution, e.g. that the operator by accident removed the arterial line from the patient to connect it according to a traditional way. To safeguard that the arterial line 102 is blood filled after the first phase, i.e. that there is no air in the arterial line 102, the controller 114 can control the apparatus 100 by driving the blood pump with the clamp 106 in a clamped state. The controller 114 can then by monitoring a pressure in the arterial line 102 by aid of a pressure sensor 128 applied at the arterial line determine compression characteristics of the content of the arterial line 102. Through Boyle's law, the pressure fluctuations in the arterial line 102 can be used to determine if there is any air in the arterial line 102, or if there is only liquid. Thus, by considering the volume aspired by the blood pump 116 and the pressure fluctuations, this determination is feasible. Consider for example if the arterial line 102 by accident was disconnected from the patient before blood restitution, and although the aspiration of a volume corresponding to the arterial line 102, which upon disconnection can aspire air, the presence of air in the arterial line 102 can be detected by this approach. This approach can also be used without the aspiration of the volume as demonstrated above. Further, the pressure fluctuation can be performed, and if no air is detected, the procedure can proceed to the second phase as demonstrated below, or if any air is detected, the procedure can proceed with the aspiration to try to remove the air, and possibly re-check with the pressure fluctuation test. If air is (still) detected, air detection alarm is of course generated, as common in the art.
During a second phase, occurring after the first phase, the controller 114 controls infusion liquid to be infused from the infusion liquid port 120 to the arterial chamber 110. This is feasible by the infusion liquid line 124 connected to the service line 126. The controller 114 further controls the blood pump 116 to drive and thereby apply a positive pressure in the arterial line 102, which has its arterial clamp 106 in a clamped state, and the arterial chamber 110. Thereby, blood is restituted through the venous line 104 to the patient. To achieve the positive pressure in the arterial line and chamber 102, 110, a higher volume of infusion liquid than or at least as high volume as aspired by the blood pump 116 is provided. The blood present in the arterial line 102 is kept there by the clamp 106 and the positive pressure. The circuit comprising the arterial chamber 110, the blood pump 116, the connection between the blood pump 116 and the blood treatment device 118, the blood treatment device 118 and the venous line 104 is thereby emptied from blood by the substitution with infusion liquid. Thus, when finally the blood of the venous line 104 is restituted, and thereby the blood of the above mentioned circuit is restituted, the procedure continues with a third phase as demonstrated below.
During the third phase, the checking whether there is any air is performed, and the procedure is ready to proceed to the fourth phase if the absence of air is ascertained. Thus, it can be noted that the temporal relation to the first and second phases of this third phase need not be in sequence, but the temporal relation between the third and fourth phase is crucial since the blood of the arterial line 102 is restituted during the fourth phase, and hence no air should be present in the arterial line 102 then. It is however beneficial to perform the checking directly before the fourth phase such that it is ascertained that no event there between causes any air to enter the arterial line. The checking can be performed by monitoring pressure fluctuations as demonstrated above.
During the fourth phase, occurring after the first, second, and third phases, the controller 114 controls the arterial clamp 106 to be in an unclamped state and the blood pump 116 to a blocked state. The blood pump 116 is thus assumed to block flow towards the blood treatment device 118. The controller then controls infusion of infusion liquid from the infusion liquid port 120 to the arterial chamber 110. Since the flow path towards the blood treatment device 118 is blocked, and the only fluid exit is through the arterial line 102, the blood in the arterial line is restituted by the corresponding infused volume. Thereby, all blood is restituted to the patient without any re-connection of lines, and there is no risk caused by the handling of the restitution process for the operator to come in contact with the patient's blood.

When restitution is finished, the patient can be disconnected.

The blood treatment apparatus 100 can comprise a User Interface, UI, 130 which is arranged to provide information to the operator and receive instructions, such as settings, operator control commands, etc., from the operator. The UI can comprise different input and output means depending on apparatus design. The input means can for example comprise dedicated keys or buttons for different functions, or physical keys or buttons can be assigned different functions depending on state of the apparatus 100, wherein the actual function preferably is indicated for the key or button. The UI 130 can also be a so called Graphical User Interface, GUI, where an increased freedom of assignment of information exchange with the operator is possible. The use of a touch screen for the GUI further enhances the information exchange with the operator, where so called soft keys can be provided with relevant keys for the current state of the apparatus 100.

The UI 130, which is controlled by the controller 114, can be arranged to enable a blood restitution key or button. This key or button is enabled when blood treatment operations are ready and the blood is to be restituted. The arrangement demonstrated above where blood restitution is controlled by the controller without any need for the operator to re-connect any lines enables a true button-click restitution feature, i.e. that the operator only interacts with the UI or GUI 130 to initiate and perform the blood restitution. If any further actions, such as ascertaining that an outlet connector from the infusion liquid port 120 is connected to the arterial service line 126, are needed to be performed by the operator, instructions thereabout can be provided through the UI or GUI 130. Thus, upon operator actuation of the blood restitution key or button, which can be a dedicated physical key or button, a physical key or button assigned the function of blood restitution based on the state of the apparatus 100, or a soft key or button provided through the GUI, possibly on a touch screen, the blood restitution procedure with the four phases is initiated and performed as demonstrated above. Thereafter, the operator can disconnect the patient.

The gist of the invention is, as demonstrated above, to provide an apparatus structure for automated blood restitution. The apparatus structure includes the controller 114 which enables the apparatus to function according to the principles demonstrated above. The designer of the controller 114, when considering the functions to be controlled, implements a method according to which the apparatus 100 is to be controlled for achieving the desired function of the apparatus 100. Fig. 4 is a flow chart illustrating such a method of control of the apparatus 100 for extracorporeal blood treatment according to an embodiment. It is ascertained 400 that arterial line 102 is blood filled.

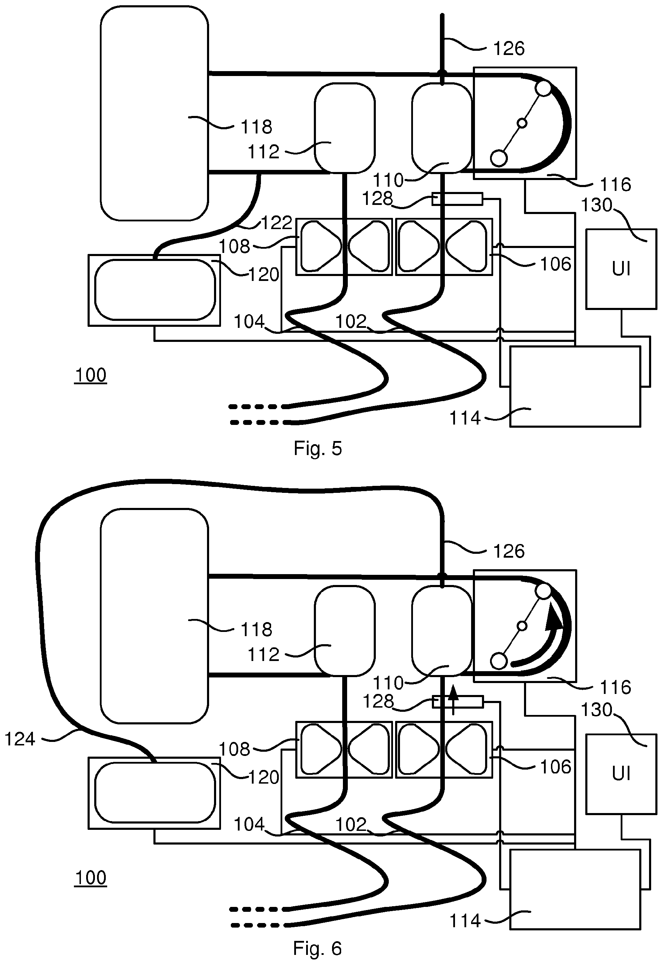

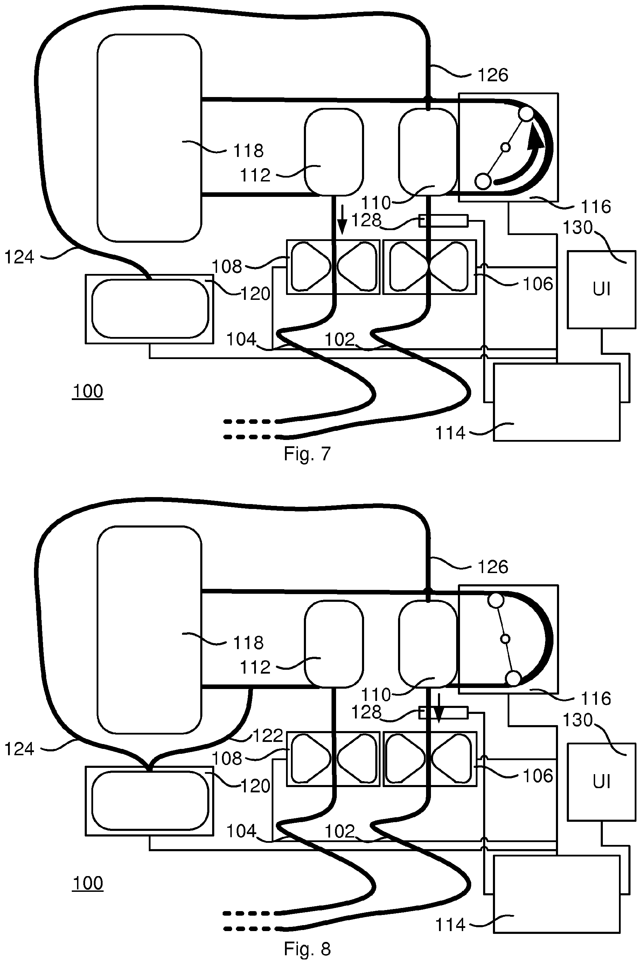

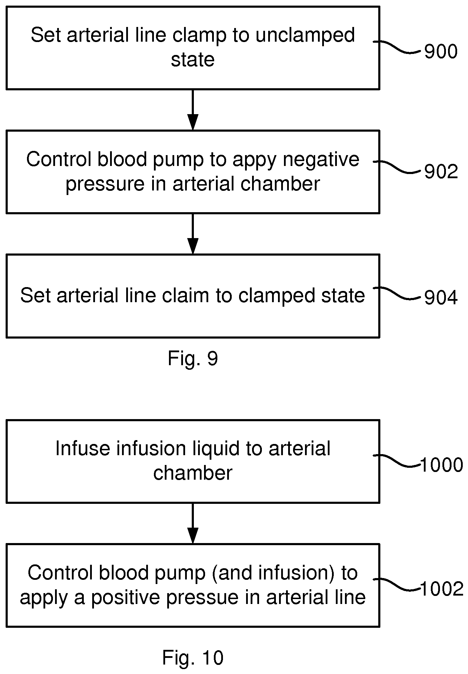

The ascertaining 400 can be performed, as illustrated in Fig. 9, by setting 900 the arterial line clamp 106 to be in an unclamped state and controlling 902 the blood pump 116 to apply a negative pressure in the arterial chamber 110, which can be performed by driving the blood pump 116 as indicated by the arrow at the blood pump 116 in Fig. 6, wherein a flow in the arterial line 102 is achieved, as depicted by the arrow at the arterial line 102 in Fig. 6. This is preferably performed such that at least a volume corresponding to the arterial line 102 is aspired. Then, the arterial line clamp 106 is set 904 to a clamped state, as depicted in Fig. 7. Other ways of ascertaining 400 can be different measuring methods, e.g. ultrasonic, where content of the arterial line 102 is monitored, or relying on information available from processes performed with the apparatus 100 prior the operations.

When it is ascertained that the arterial line 102 is blood filled, i.e. free from air, and clamped by the arterial line clamp 106, the controller causes emptying 402 of blood of the venous line 104. Since, as demonstrated above with reference to the apparatus 100, a circuit is provided from the arterial chamber 110, via the blood pump 116, which is driven as indicated by the arrow at the blood pump 116 in Fig. 7, the blood treatment device 118 and the venous chamber 112, to the arterial line 104, infusion liquid inserted into the arterial chamber 110 through a service line 126 can be used to push blood of this circuit out via the venous line 104, as depicted by the arrow at the venous line 104 in Fig. 7, and finally also empty the venous line 104. Thus, the controller 114 controls the infusion 1000 and controls 1002 the blood pump 116, as depicted in Fig. 10, to push the liquid as explained above. The relation between the controlling 1000 of the infusion and the controlling 1002 of the blood pump is such that a positive pressure in the arterial chamber 110 is maintained. A positive pressure of about 50 mmHg is considered appropriate. The pressure sensor 128 on the arterial line 102 can provide a signal that can be used for feedback of the control. One way of determining when the venous line 104 is emptied from blood is that the controller 114 knows the volume of the circuit mentioned above and by its control of the blood pump 116 knows the volume that has been provided. Another way of determining when the venous line is emptied from blood is to measure, e.g. optically or electrically, at a predetermined position in the circuit, wherein it is determined that no blood is present at that position, and by knowing the volume from that position to the end of the venous line control the blood pump 116 to finish the emptying 402. When the controller 114 considers the venous line 104 as emptied from blood, it can proceed by emptying 406 blood from the arterial line 102. At a time instant at least before the arterial line emptying procedure 406, the controller also makes a check to ascertain 404 that no air is present in the arterial line 102. The ascertaining 404 can for example be performed after the venous line 102 is emptied 402 and thus just before the commence of the emptying 406 of the arterial line to ascertain that no air has happened to enter the arterial line during the other operation steps. This can be performed by air detection measurement, e.g. ultrasonic. However, since lines of the apparatus 100 are exchanged between treatments, sensors integrated with the line package must not be too costly, and thus implying that signal quality, i.e. reliability of measurement, may not be satisfactory for a reasonably priced line package. Sensors to measure through an exchangeable line package also have their problems due to physical constraints in interaction between exchangeable line package and sensor. An advantageous approach for determining whether any air is present in the arterial line can be provided by the structure of the apparatus 100, as demonstrated above. The controller 114 keeps 1100 the arterial line clamp 106 in a clamped state and measures 1102 the pressure in the arterial line 102, e.g. by the pressure sensor 128. The controller 114 then controls 1104 the blood pump 116 to aspire a predetermined volume. The pressure in the arterial line 102 is again measured 1106, and from the aspired volume and the pressure measurements can be used by the controller 114 to determine whether there is any air present in the arterial line 102. For this, Boyle's law, which states that the absolute pressure and volume of a given mass of confined gas are inversely proportional, if the temperature remains unchanged within a closed system, can be used to determine if there is any confined gas, i.e. air in the arterial line. Thereby, no sensor elements need to be included for air sensing in the exchangeable lines or any complex arrangements for sensors to be arranged at the exchangeable lines when inserting them into the apparatus 100.

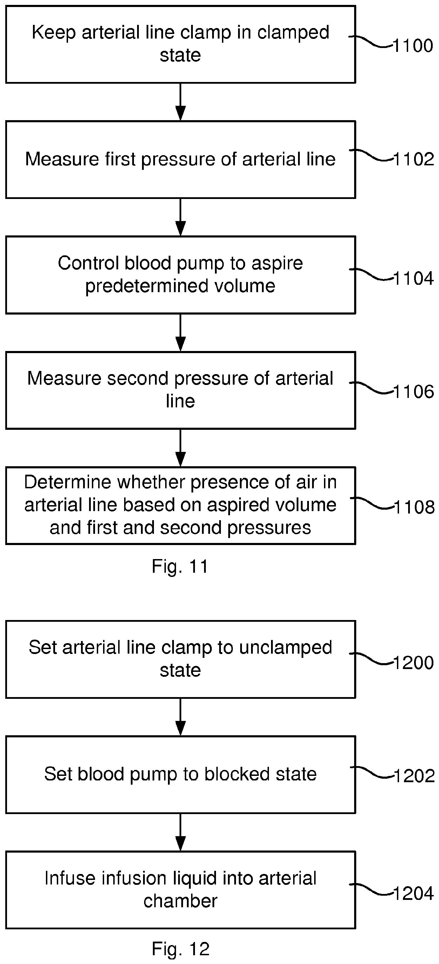

The emptying 406 of the arterial line 102 includes, as depicted in Fig. 12, setting 1200 the arterial line clamp 106 to an unclamped state and setting 1202 the blood pump 116 to a blocked state, as depicted in Fig. 8. The controller 114 then controls infusing 1204 of infusion liquid into the arterial chamber 110. The infusion liquid thereby pushes blood of the arterial line 102, as indicated by the arrow in Fig. 8, such that it is emptied 406 from blood, and blood is thereby substantially emptied from the lines of the apparatus 100. Thereby, the lines of e.g. a line package can substantially be emptied from blood.

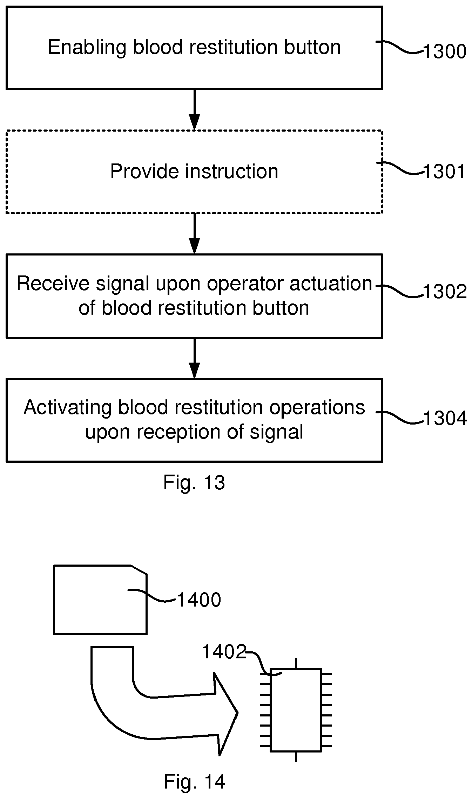

The controller 114 can also be arranged to control and interact with the UI 130. When it is time to empty the lines from blood, i.e. after blood treatment, the controller 114 enables 1300 a blood restitution key or button on the UI 130, as depicted in Fig. 13. The enabling 1300 can, as demonstrated above with reference to the apparatus 100, be enabling of a dedicated key or button, etc. When the UI 130 is provided as a GUI on a screen, e.g. a touch screen, enabling 1300 includes presenting the blood restitution key or button as a soft key or button on the screen. The controller 114 can then receive 1302 a signal from the UI upon operator actuation of the blood restitution key or button, wherein the controller 114 upon reception of the signal activates 1304 the operations 400, 402, 404, 406 for emptying the lines. Further optionally, the controller 114 and the UI 130 can provide instructions through a screen of the UI 130 to an operator to connect an outlet connector from the infusion liquid port 120 to the service line 126. This instruction can be performed when the blood restitution key or button is enabled, possibly based also on a sensor input providing a signal whether the service line 126 already is connected to the liquid port 120 or not.

The methods according to the present invention is suitable for implementation with aid of processing means, such as computers and/or processors, especially for the case where the controller 114 of the apparatus 100 for extracorporeal blood treatment is a digital controller including a processing means, processor, or computer performing its actions based on a computer program. Therefore, there is provided computer programs, comprising instructions arranged to cause the processing means, processor, or computer to perform the steps of any of the methods according to any of the embodiments described with reference to Figs 4 and 9 to 13. The computer programs preferably comprises program code which is stored on a computer readable medium 1400, as illustrated in Fig. 14, which can be loaded and executed by a processing means, processor, or computer 1402 to cause it to perform the methods, respectively, according to embodiments of the present invention, preferably as any of the embodiments described with reference to Figs 4 and 9 to 13. The computer 1402 and computer program product 1400 can be arranged to execute the program code sequentially where actions of the any of the methods are performed stepwise. The processing means, processor, or computer 1402 is preferably what normally is referred to as an embedded system. Thus, the depicted computer readable medium 1400 and computer 1402 in Fig. 14 should be construed to be for illustrative purposes only to provide understanding of the principle, and not to be construed as any direct illustration of the elements.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A blood treatment apparatus (100) for extracorporeal blood treatment comprising
an arterial line (102) and a venous line (104) for connecting to a patient, wherein an arterial clamp (106) is arranged on the arterial line (102) to selectably clamp and unclamp the arterial line (102);
an arterial chamber (110) connected to the arterial line;
a venous chamber (112) connected to the venous line;
a blood pump (116) connected to the arterial chamber (110) and arranged to provide blood from the arterial chamber via a blood treatment device (118) towards the venous chamber (112);
an infusion liquid port (120) for connection of a infusion liquid source; and
a controller (114) arranged to, at blood restitution operation,
control a first phase of the blood restitution operation including control operations to ascertain that the arterial line (102) is filled with blood, and then to set the arterial line clamp (106) to a clamped state wherein the blood restitution operation is enabled to proceed to a second phase of the blood restitution operation;
control the second phase of the blood restitution operation including control operations to
infuse liquid from the infusion liquid port (120) to the arterial chamber (110), and
control the blood pump (116) together with the supplied infusion to apply a positive pressure in the arterial line (102) while the arterial line clamp (106) is controlled to be in a clamped state such that blood is restituted, by drive control of the blood pump (116), to the patient via the venous line (104),
wherein the blood restitution operation is enabled to proceed to a third phase of the blood restitution operation when the blood of the venous line (104) has been restituted to the patient;
control the third phase of the blood restitution operation including control operations to ascertain, by monitoring, that no air is present in the arterial line (102), wherein the blood restitution operation is enabled to proceed to a fourth phase of the blood restitution operation when it is ascertained that no air is present in the arterial line (102);
control the fourth phase of the blood restitution operation including control operations to
set the arterial line clamp (106) to an unclamped state, and set the blood pump (116) to a blocked state, and then
infuse infusion liquid into the arterial chamber (110) such that blood in the arterial line (102) is restituted to the patient.

2. The apparatus (100) according to claim 1, wherein the control operation to ascertain that the arterial line (102) is filled with blood includes to
initially set the arterial line clamp (106) to be in unclamped state, and then
control the blood pump (116) to apply a negative pressure in the arterial chamber (110) such that a volume of the arterial line (102) is aspired, and then
set the arterial line clamp (106) to a clamped state.

3. The apparatus (100) according to claim 1 or 2, wherein control operations to ascertain that no air is present in the arterial line (102) includes determination of compression characteristics of the arterial line (102) by a test, with the arterial line clamp (106) in a clamped state, by application of a pressure in the arterial line (102) by control of the blood pump (116) and monitoring of the pressure in the arterial line (102) by a pressure sensor (128).

4. The apparatus (100) according to claim 3, wherein the monitoring of the pressure in the arterial line (102) includes a pressure sample before and a pressure sample after changing the volume of the arterial chamber (110) by a predetermined volume aspired by the controlling of the blood pump (116), and applying Boyle's law for determining whether any air is present in the arterial line (102).

5. The apparatus (100) according to any of claims 1 to 4, wherein infusion of liquid to the arterial chamber (110) comprises to control an infusion pump, by the controller (114), to provide the infusion liquid into the arterial chamber (110) via a service line (126) connected to the arterial chamber (110).

6. The apparatus (100) according to any of claims 1 to 5, comprising a user interface, UI, (130) controlled by the controller (114), wherein the UI (130) is arranged to enable a blood restitution button when blood treatment operations are ready, and upon operator actuation of the blood restitution button, the controller (114) is configured to activate the blood restitution operation.

7. The apparatus (100) according to claim 6, wherein the UI (130) is arranged to, when the blood restitution button is enabled, provide an instruction to the operator to connect an outlet connector from an infusion liquid port to an arterial service line being in connection with the arterial chamber.

8. The apparatus (100) according to claim 6 or 7, wherein the UI (130) comprises a touchscreen, and the blood restitution button is a soft button presented on the touch screen.

9. The apparatus (100) according to any of claims 1 to 8, wherein the apparatus (100) is a hemodialysis apparatus, and the blood treatment device (118) is a dialyser.

10. A computer program comprising program code means for performing all the steps of a method of controlling a blood treatment apparatus for extracorporeal blood treatment when said program is run on a processor (1402) of a controller of a blood treatment apparatus for extracorporeal blood treatment, the apparatus comprising an arterial line and a venous line for connecting to a patient, wherein an arterial clamp is arranged on the arterial line for selectable clamping and unclamping of the arterial line, an arterial chamber for connecting to the arterial line, a venous chamber for connecting to the venous line, a blood pump connected to the arterial chamber and for providing blood via a blood treatment device towards the venous chamber, and an infusion liquid port for connection of a infusion liquid source, the method comprising
controlling a first phase of operation comprising
ascertaining (400) that the arterial line has been filled with blood, and then
setting the arterial line clamp to a clamped state, and then
enabling the operation to proceed to a second phase of the operation;
controlling the second phase of the blood restitution operation comprising
infusing (1000) infusion liquid from the infusion liquid port to the arterial chamber, and
controlling (1002) the blood pump together with the supplied infusion liquid to apply a positive pressure in the arterial line while the arterial line clamp is in a clamped state such that a liquid flow is achieved (402), by driving the blood pump, through the venous line,
enabling the operation to proceed to a third phase of the operation when the blood of the venous line is emptied;
controlling the third phase of the operation comprising
ascertaining (404), by monitoring, that no air is present in the arterial line, and
enabling the operation to proceed to a fourth phase of the operation when it is ascertained that no air is present in the arterial line;
controlling the fourth phase of the operation comprising
setting (1200) the arterial line clamp to an unclamped state, and
setting (1202) the blood pump to a blocked state, and then infusing (1204) infusion liquid into the arterial chamber such that blood in the arterial line is emptied (406).

11. The program according to claim 10, wherein the ascertaining (400) that the arterial line is filled with blood comprises
initially setting (900) the arterial line clamp to be in unclamped state, and then
controlling (902) the blood pump to apply a negative pressure in the arterial chamber such that a volume of the arterial line is aspired, and then
setting (904) the arterial line clamp to a clamped state.

12. The program according to claim 10 or 11, wherein the ascertaining (404) that no air is present in the arterial line comprises
determining (1108) compression characteristics of the arterial line by a test, with the arterial line clamp in a clamped state, by applying (1104) a pressure in the arterial line by controlling the blood pump and monitoring the pressure in the arterial line.

13. The program according to claim 12, wherein the monitoring of the pressure in the arterial line is performed before (1102) and after (1106) changing (1104) the volume of the arterial chamber by a predetermined volume aspired by the controlling of the blood pump, and the determining (1108) comprises applying Boyle's law for determining whether any air is present in the arterial line.

14. The program according to any of claims 10 to 13, wherein infusing (1000) liquid to the arterial chamber comprises providing the infusion liquid into the arterial chamber via a service line connected to the arterial chamber.

15. The program according to any of claims 10 to 14, wherein the apparatus comprises a user interface, UI, and the method further comprises
enabling (1300) a blood restitution button on the UI when blood treatment operations are ready,
receiving (1302) a signal upon operator actuation of the blood restitution button, and
activating (1304), upon reception of the signal, the controlling of the operations (400, 402, 404, 406).

16. The program according to claim 15, further comprising
providing (1301) an instruction through the UI, when the blood restitution button is enabled (1300), to the operator to connect an outlet connector from an infusion liquid port to an arterial service line being in connection with the arterial chamber.

17. The program according to claim 15 or 16, wherein the UI comprises a screen, and the enabling (1300) of the blood restitution button comprises presenting the blood restitution button as a soft button on the screen.

## Patentansprüche

1. Blutbehandlungsvorrichtung (100) zur extrakorporalen Blutbehandlung, umfassend:
eine arterielle Leitung (102) und eine venöse Leitung (104) zur Verbindung mit einem Patienten, wobei eine arterielle Klemme (106) an der arterielle Leitung (102) angeordnet ist zum selektiven Abklemmen und Öffnen der arteriellen Leitung (102);
eine arterielle Kammer (110), die mit der arteriellen Leitung verbunden ist;
eine venöse Kammer (112), die mit der venösen Leitung verbunden ist;
eine Blutpumpe (116), die mit der arteriellen Kammer (110) verbunden und angeordnet ist, Blut von der arteriellen Kammer durch eine Blutbehandlungseinrichtung (118) zur venösen Kammer (112) zuzuführen;
einen Infusionsflüssigkeitsanschluss (120) zur Verbindung einer Infusionsflüssigkeitsquelle; und
eine Steuereinheit (114), die angeordnet ist, im Blutrückgabebetrieb, zum
Steuern eines ersten Schrittes des Blutrückgabebetriebs umfassend Steueroperationen zum Ermitteln, ob die arterielle Leitung (102) mit Blut gefüllt ist, und dann zum Einstellen der arteriellen Leitungsklemme (106) in einen abgeklemmten Zustand, wobei der Blutrückgabebetrieb dazu freigegeben wird, einen zweiten Schritt des Blutrückgabebetriebs auszuführen;
Steuern des zweiten Schrittes des Blutrückgabebetriebs umfassend Steueroperationen zum
Infundieren von Flüssigkeit vom Infusionsflüssigkeitsanschluss (102) in die arterielle Kammer (110), und
Steuern der Blutpumpe (116) zusammen mit der zugeführten Infusion, um einen positiven Druck in der arteriellen Leitung (102) anzuwenden, wobei die arterielle Leitungsklemme (106) gesteuert wird, in einem abgeklemmten Zustand zu sein, so dass das Blut durch Betätigung der Blutpumpe (116) dem Patienten durch die venöse Leitung (104) rückgegeben wird,
wobei der Blutrückgabebetrieb freigegeben wird, einen dritten Schritt des Blutrückgabebetriebs auszuführen, wenn das Blut von der venösen Leitung (104) dem Patienten zurückgegeben worden ist;
Steuern des dritten Schrittes des Blutrückgabebetriebs umfassend Steueroperationen zum Ermitteln, durch Überwachen, dass keine Luft in der arteriellen Leitung (102) vorhanden ist, wobei der Blutrückgabebetrieb dazu freigegeben wird, einen vierten Schritt des Blutrückgabebetriebs auszuführen, wenn ermittelt worden ist, dass keine Luft in der arteriellen Leitung (102) vorhanden ist;
Steuern des vierten Schrittes des Blutrückgabebetriebs umfassend Steueroperationen zum
Einstellen der arteriellen Leitungsklemme (106) in einen geöffneten Zustand, und
Einstellen der Blutpumpe (116) in einen verriegelten Zustand, und dann
Infundieren von Infusionsflüssigkeit in die arterielle Kammer (110), so dass das Blut in der arteriellen Leitung (102) dem Patienten zurückgegeben wird.

2. Vorrichtung (100) nach Anspruch 1, wobei die Steueroperation zum Ermitteln, ob die arterielle Leitung (102) mit Blut gefüllt ist, beinhaltet:
anfängliches Einstellen der arteriellen Leitungsklemme (106) in einen geöffneten Zustand, und dann
Steuern der Blutpumpe (116), um einen negativen Druck in der arteriellen Kammer (110) anzuwenden, so dass ein Volumen der arteriellen Leitung (102) angesaugt wird, und dann
Einstellen der arteriellen Leitungsklemme (106) in einen abgeklemmten Zustand.

3. Vorrichtung (100) nach Anspruch 1 oder 2, wobei die Steueroperationen zum Ermitteln, dass keine Luft in der arteriellen Leitung (102) vorhanden ist, Bestimmen der Druckeigenschaften in der arteriellen Leitung (102) durch ein Test beinhaltet, wobei die arterielle Leitungsklemme (106) in einem abgeklemmten Zustand ist, durch Anwenden eines Druckes in der arteriellen Leitung (102) durch Steuern der Blutpumpe (116) und Überwachen des Druckes in der arteriellen Leitung (102) mittels einem Drucksensor (128).

4. Vorrichtung (100) nach Anspruch 3, wobei das Überwachen des Druckes in der arteriellen Leitung (102) eine Druckprobe vor und eine Druckprobe nach Volumenänderung in der arteriellen Kammer (110) um ein vorbestimmtes Volumen durch Steuern der Blutpumpe (116) und Anwenden des Boyleschen Gesetzes zum Bestimmen, ob Luft in der arteriellen Leitung (102) vorhanden ist, beinhaltet.

5. Vorrichtung (100) nach einem der Ansprüche 1 bis 4, wobei das Infundieren der Flüssigkeit in die arterielle Kammer (110) das Steuern einer Infusionspumpe durch die Steuereinheit (114) umfasst, um die Infusionsflüssigkeit in die arterielle Kammer (110) durch eine mit der arteriellen Kammer (110) verbundene Serviceleitung (126) zuzuführen.

6. Vorrichtung (100) nach einem der Ansprüche 1 bis 5, umfassend eine Benutzerschnittstelle UI (130), die von der Steuereinheit (114) gesteuert wird, wobei die UI (130) so angeordnet ist, einen Blutrückgabeknopf freizugeben, wenn die Blutbehandlungsoperationen fertig sind, und beim Betätigen des Blutrückgabeknopfes vom Benutzer ist die Steuereinheit konfiguriert zum Aktivieren des Blutrückgabebetriebs.

7. Vorrichtung (100) nach Anspruch 6, wobei die UI (130) so ausgestaltet ist, eine Anweisung zum Benutzer zu senden, wenn der Blutrückgabeknopf freigegeben wird, um einen Ausgangsverbinder von einem Infusionsflüssigkeitsanschluss mit einer arteriellen Serviceleitung zu verbinden, die mit der arteriellen Kammer verbunden ist.

8. Vorrichtung (100) nach Anspruch 6 oder 7, wobei die UI (130) einen Berührungsbildschirm umfasst und der Blutrückgabeknopf einen auf dem Berührungsbildschirm angezeigten Soft-Knopf umfasst.

9. Vorrichtung (100) nach einem der Ansprüche 1 bis 8, wobei die Vorrichtung (100) eine Hämodialysevorrichtung ist und die Blutbehandlungseinrichtung (118) ein Dialysegerät ist.

10. Computerprogramm umfassend Programmcodemittel zur Ausführung aller Schritte eines Verfahrens zum Steuern einer Blutbehandlungsvorrichtung zur extrakorporalen Blutbehandlung, wenn das Programm auf einem Prozessor (1402) einer Steuereinheit einer Blutbehandlungsvorrichtung zur extrakorporalen Blutbehandlung ausgeführt wird, die Vorrichtung umfassend eine arterielle Leitung und eine venöse Leitung zum Verbinden mit einem Patienten, wobei eine arterielle Klemme an der arterielle Leitung zum selektiven Abklemmen und Öffnen der arteriellen Leitung angeordnet ist, eine arterielle Kammer zum Verbinden mit der arteriellen Leitung, eine venöse Kammer zum Verbinden mit der venösen Leitung, eine Blutpumpe, die mit der arteriellen Kammer verbunden ist zum Zuführen von Blut durch eine Blutbehandlungseinrichtung zur venösen Kammer, und einen Infusionsflüssigkeitsanschluss zur Verbindung einer Infusionsflüssigkeitsquelle, das Verfahren umfassend
Steuern eines ersten Betriebsschrittes umfassend
Ermitteln (400), ob die arterielle Leitung mit Blut gefüllt worden ist, und dann
Einstellen der arteriellen Leitungsklemme in einen abgeklemmten Zustand, und dann
Freigeben des Betriebs, einen zweiten Schritt des Betriebs auszuführen;
Steuern des zweiten Schrittes des Blutrückgabebetriebs umfassend
Infundieren (1000) von Infusionsflüssigkeit vom Infusionsflüssigkeitsanschluss in die arterielle Kammer, und
Steuern (1002) der Blutpumpe zusammen mit der zugeführten Infusionsflüssigkeit, um einen positiven Druck in der arteriellen Leitung anzuwenden, während die arterielle Leitungsklemme in einem abgeklemmten Zustand ist, so dass ein Flüssigkeitsfluss durch die venöse Leitung mittels Betätigung der Blutpumpe erreicht wird (402),
Freigeben des Betriebs, einen dritten Schritt des Betriebs auszuführen, wenn das Blut der venösen Leitung entleert wird;
Steuern des dritten Schrittes des Betriebs umfassend
Ermitteln (404) durch Überwachen, dass keine Luft in der arteriellen Leitung vorhanden ist,
Freigeben des Betriebs, einen vierten Schritt des Betriebs auszuführen, wenn ermittelt wird, dass keine Luft in der arteriellen Leitung vorhanden ist;
Steuern des vierten Schrittes des Betriebs umfassend
Einstellen (1200) der arteriellen Leitungsklemme in einen geöffneten Zustand, und
Einstellen (1202) der Blutpumpe in einen verriegelten Zustand, und dann
Infundieren (1204) von Infusionsflüssigkeit in die arterielle Kammer, so dass das Blut in der arteriellen Leitung entleert wird (406).

11. Programm nach Anspruch 10, wobei das Ermitteln (400), dass die arterielle Leitung mit Blut gefüllt ist,
anfängliches Einstellen (900) der arteriellen Leitungsklemme in einen geöffneten Zustand, und dann
Steuern (902) der Blutpumpe, um einen negativen Druck in der arteriellen Kammer anzuwenden, so dass ein Volumen der arteriellen Leitung angesaugt wird, und dann
Einstellen (904) der arteriellen Leitungsklemme in einen abgeklemmten Zustand, umfasst.

12. Programm nach Anspruch 10 oder 11, wobei das Ermitteln (404), dass keine Luft in der arteriellen Leitung vorhanden ist, umfasst
Bestimmen (1108) der Druckeigenschaften in der arteriellen Leitung durch ein Test, wobei die arterielle Leitungsklemme in einem abgeklemmten Zustand ist, durch Anwenden (1104) eines Druckes in der arteriellen Leitung durch Steuern der Blutpumpe und Überwachen des Druckes in der arteriellen Leitung.

13. Programm nach Anspruch 12, wobei das Überwachen des Druckes in der arteriellen Leitung vor (1102) und nach (1106) der Volumenänderung (1104) in der arteriellen Kammer um ein vorbestimmtes, angesaugtes Volumen durch Steuern der Blutpumpe ausgeführt wird, und das Bestimmen (1108) das Anwenden des Boyleschen Gesetzes zum Bestimmen, ob Luft in der arteriellen Leitung vorhanden ist, umfasst.

14. Programm nach einem der Ansprüche 10 bis 13, wobei das Infundieren (1000) der Flüssigkeit in die arterielle Kammer das Zuführen der Infusionsflüssigkeit in die arterielle Kammer durch eine mit der arteriellen Kammer verbundene Serviceleitung umfasst.

15. Programm nach einem der Ansprüche 10 bis 14, wobei die Vorrichtung eine Benutzerschnittstelle UI umfasst und das Verfahren weiter umfasst
Freigeben (1300) eines Blutrückgabeknopfes auf der UI, wenn die Blutbehandlungsoperationen fertig sind,
Empfangen (1302) eines Signals beim Betätigen des Blutrückgabeknopfes durch den Benutzer, und
Aktivierung (1304), beim Empfang des Signals, der Steuern des Betriebs (400, 402, 404, 406).

16. Programm nach Anspruch 15, weiter umfassend Zuführen (1301) einer Anweisung durch die UI, wenn der Blutrückgabeknopf freigegeben wird (1300), dem Benutzer, um einen Ausgangsverbinder von einem Infusionsflüssigkeitsanschluss mit einer arteriellen Serviceleitung zu verbinden, die mit der arteriellen Kammer verbunden ist.

17. Programm nach Anspruch 15 oder 16, wobei die UI einen Bildschirm umfasst und das Freigeben (1300) des Blutrückgabeknopfes die Anzeige des Blutrückgabeknopfes als Soft-Knopf auf dem Schirm umfasst.

## Revendications

1. Appareil de traitement du sang (100) pour le traitement extracorporel du sang, comprenant:
un conduit artériel (102) et un conduit veineux (104) à relier à un patient, où une pince artériel (106) est rangé sur le conduit artériel (102) pour serrer et desserrer sélectivement le conduit artériel (102);
un compartiment artériel (110) relié au conduit artériel;
un compartiment veineux (112) relié au conduit veineux;
une pompe du sang (116) reliée au compartiment artériel (110) et apte à porter le sang du compartiment artériel au moyen d'un dispositif de traitement du sang (118) vers le compartiment veineux (112);
une porte du liquide d'infusion (120) pour relier une source de liquide d'infusion; et
une unité de commande (114) apte à, pendant l'opération de restitution du sang,
commander une première étape de l'opération de restitution du sang comprenant des opérations de contrôle pour vérifier que le conduit artériel (102) est rempli de sang, et ensuite pour régler la pince artériel (106) dans un état serré où l'opération de restitution du sang est autorisée à passer à une deuxième étape de l'opération de restitution du sang;
commander la deuxième étape de l'opération de restitution du sang comprenant des opérations de contrôle pour
infuser du liquide de la porte du liquide d'infusion (120) au compartiment artériel (110), et
commander la pompe du sang (116) avec l'infusion réalisée pour appliquer une pression positive dans le conduit artériel (102), tandis que la pince artériel (106) est commandé pour être dans un état serré de sorte que le sang soit restitué, en actionnant la pompe du sang (116), au patient à travers le conduit veineux (104),
où l'opération de restitution du sang est autorisée à passer à une troisième étape de l'opération de restitution du sang lorsque le sang du conduit veineux (104) a été restitué au patient;
commander la troisième étape de l'opération de restitution du sang comprenant des opérations de contrôle pour vérifier par surveillance qu'il n'y a pas d'air dans le conduit artériel (102), où l'opération de restitution du sang est autorisée à passer à une quatrième étape de l'opération de restitution du sang lorsqu'il est vérifié qu'il n'y a pas d'air dans le conduit artériel (102);
commander la quatrième étape de l'opération de restitution du sang comprenant des opérations de contrôle pour
régler la pince artériel (106) dans un état ouvert, et
régler la pompe du sang (116) dans un état bloqué, et ensuite
infuser du liquide d'infusion dans le compartiment artériel (102) de sorte que le sang dans le conduit artériel (102) est restitué au patient.

2. Appareil (100) selon la revendication 1, où l'opération de contrôle pour vérifier que le conduit artériel (102) est rempli de sang comprend
régler initialement la pince artériel (106) dans un état ouvert, et ensuite
commander la pompe du sang (116) pour appliquer une pression négative dans le compartiment artériel (110) de sorte qu'un volume du conduit artériel (102) soit aspiré, et ensuite
régler la pince artériel (106) dans un état serré.

3. Appareil (100) selon la revendication 1 ou 2, où les opérations de contrôle pour vérifier qu'il n'ya pas d'air dans le conduit artériel (102) comprennent la détermination des caractéristiques de compression du conduit artériel (102) par un test, avec la pince artériel (106) dans un état serré, en appliquant une pression dans le conduit artériel (102) par commande de la pompe du sang (116) et surveillance de la pression dans le conduit artériel (102) au moyen d'un capteur de pression (128).

4. Appareil (100) selon la revendication 3, où la surveillance de la pression dans le conduit artériel (102) comprend un échantillonnage de la pression avant et un échantillonnage après la variation de volume dans le compartiment artériel (110) d'un volume préétabli aspiré, en commandant la pompe du sang (116) et en appliquant la loi de Boyle pour déterminer s'il y a de l'air dans le conduit artériel (102).

5. Appareil (100) selon l'une quelconque des revendications 1 à 4, où l'infusion de liquide dans le compartiment artériel (110) comprend commander une pompe d'infusion au moyen de l'unité de commande (114) pour introduire le liquide d'infusion dans le compartiment artériel (110) par un conduit de service (126) relié au compartiment artériel (110).

6. Appareil (100) selon l'une quelconque des revendications 1 à 5, comprenant une interface utilisateur IU (130) commandée par l'unité de commande (114), où l'IU (130) est apte à activer un bouton de restitution du sang lorsque les opérations de traitement du sang sont terminées, et une fois activé le bouton de restitution du sang par un opérateur, l'unité de commande (114) est configurée pour activer l'opération de restitution du sang.

7. Appareil (100) selon la revendication 6, où l'IU (130) est apte à, lorsque le bouton de restitution du sang est activé, envoyer une instruction à l'opérateur pour relier un connecteur de sortie d'une porte du liquide d'infusion à un conduit de service artériel relié au compartiment artériel.

8. Appareil (100) selon la revendication 6 ou 7, où l'IU (130) comprend un écran tactile et le bouton de restitution du sang est une touche affichée sur l'écran tactile.

9. Appareil (100) selon l'une quelconque des revendications 1 à 8, où l'appareil (100) est un appareil d'hémodialyse et le dispositif de traitement du sang (118) est un dialyseur.

10. Programme pour ordinateur comprenant des moyens d'encodage de programmes pour exécuter toutes les étapes d'un procédé pour commander un appareil de traitement du sang pour le traitement extracorporel du sang lorsque ledit programme est exécuté dans un processeur (1402) d'une unité de commande d'un appareil de traitement du sang pour le traitement extracorporel du sang, l'appareil comprenant un conduit artériel et un conduit veineux apte à être reliés à un patient, où une pince artériel est rangé sur le conduit artériel pour serrer et desserrer sélectivement le conduit artériel, un compartiment artériel apte à être relié au conduit artériel, un compartiment veineux apte à être relié au conduit veineux, une pompe du sang reliée au compartiment artériel et apte à porter le sang au moyen d'un dispositif de traitement du sang vers le compartiment veineux, et une porte du liquide d'infusion pour relier une source de liquide d'infusion, le procédé comprenant
commander une première étape de l'opération comprenant
vérifier (400) que le conduit artériel a été rempli de sang, et ensuite
régler la pince artériel dans un état serré, et ensuite
autoriser l'opération à passer à une deuxième étape de l'opération;
commander la deuxième étape de l'opération de restitution du sang comprenant
infuser (1000) du liquide d'infusion de la porte du liquide d'infusion au compartiment artériel, et
commander (1002) la pompe du sang avec le liquide d'infusion pour appliquer une pression positive dans le conduit artériel, tandis que la pince artériel est dans un état serré de sorte à atteindre un flux de liquide (402), en actionnant la pompe du sang, à travers le conduit veineux,
autoriser l'opération à passer à une troisième étape de l'opération lorsque le conduit veineux est vide;
commander la troisième étape de l'opération comprenant
vérifier (404) par surveillance qu'il n'y a pas d'air dans le conduit artériel (102), et
autoriser l'opération à passer à une quatrième étape de l'opération lorsqu'il est vérifié qu'il n'y a pas d'air dans le conduit artériel;
commander la quatrième étape de l'opération comprenant
régler (1200) la pince artériel dans un état ouvert, et ensuite
régler (1202) la pompe du sang dans un état bloqué, et ensuite
infuser (1204) du liquide d'infusion dans le compartiment artériel de sorte que le sang dans le conduit artériel est vidé.

11. Programme selon la revendication 10, où vérifier (400) que le conduit artériel est rempli de sang comprend
régler initialement (900) la pince du conduit artériel dans un état ouvert, et ensuite
commander (902) la pompe du sang pour appliquer une pression négative dans le compartiment artériel de sorte à aspirer un volume du conduit artériel, et ensuite
régler (904) la pince du conduit artériel dans un état serré.

12. Programme selon la revendication 10 ou 11, où vérifier (404) qu'il n'y a pas d'air dans le compartiment artériel comprend
déterminer (1108) les caractéristiques de compression du conduit artériel par un test, avec la pince du conduit artériel dans un état serré, en appliquant (1104) une pression dans le conduit artériel par la commande de la pompe du sang et la surveillance de la pression dans le conduit artériel.

13. Programme selon la revendication 12, où la surveillance de la pression dans le conduit artériel est exécutée avant (1102) et après (1106) la variation (1104) du volume du compartiment artériel d'un volume préétabli aspiré par la commande de la pompe du sang, et la détermination (1108) comprend l'application de la loi de Boyle pour déterminer s'il y a de l'air dans le compartiment artériel.

14. Programme selon l'une quelconque des revendications 10 à 13, où infuser (1000) du liquide dans le compartiment artériel comprend introduire le liquide d'infusion dans le compartiment artériel par un conduit de service relié au compartiment artériel.

15. Programme selon l'une quelconque des revendications 10 à 14, où l'appareil comprend une interface utilisateur IU et le procédé comprend en outre
activer (1300) un bouton de restitution du sang sur l'IU lorsque les opérations de traitement du sang sont terminées,
recevoir (1302) un signal une fois que l'opérateur a actionné le bouton de restitution du sang, et
activer (1304), une fois reçu le signal, le contrôle des opérations (400, 402, 404, 406).

16. Programme selon la revendication 15, comprenant en outre
envoyer (1301) une instruction à travers l'IU, lorsque le bouton de restitution du sang est activé (1300), à l'opérateur pour relier un connecteur de sortie d'une porte du liquide d'infusion à un conduit de service artériel relié au compartiment artériel.

17. Programme selon la revendication 15 ou 16, où l'IU comprend un écran et l'activation (1300) du bouton de restitution du sang comprend afficher le bouton de restitution du sang en tant que touche sur l'écran.
